# EUROPÄISCHE PATENTSCHRIFT

(11) **EP 2 049 680 B1**
(45) Veröffentlichungstag und Bekanntmachung des Hinweises auf die Patenterteilung: **29.12.2010**
(21) Anmeldenummer: 07765027.3
(22) Anmeldetag: 03.07.2007
(51) Int. Cl.: C12Q 1/68

(54) **VERFAHREN UND VORRICHTUNG ZUR AUTHENTIFIZIERUNG VON MIT EINER MARKIERUNG VERSEHENEN GEGENSTÄNDEN**
METHOD AND DEVICE FOR AUTHENTICATING OBJECTS PROVIDED WITH A MARKER
PROCÉDÉ ET DISPOSITIF POUR AUTHENTIFIER DES OBJETS MUNIS D'UN REPÈRE

(30) Priorität: 03.07.2006 DE 102006031015
(43) Veröffentlichungstag der Anmeldung: 22.04.2009
(73) Patentinhaber: Secutech International Pte. Ltd., Singapore 069443 (SG)
(72) Erfinder: JOSTEN, André, 90429 Nürnberg (DE); WOLFRUM, Christian, 91052 Erlangen (DE)
(74) Vertreter: UEXKÜLL & STOLBERG
(86) Internationale Anmeldenummer: PCT/EP2007/005884
(87) Internationale Veröffentlichungsnummer: WO 2008/014861

(56) Entgegenhaltungen:
- WO-A-01/51652
- WO-A-02/072878
- WO-A-03/093797
- GB-A- 1 195 531
- DE SUTTER VALERIE ET AL: "Exploration of jasmonate signalling via automated and standardized transient expression assays in tobacco cells." THE PLANT JOURNAL : FOR CELL AND MOLECULAR BIOLOGY DEC 2005, Bd. 44, Nr. 6, Dezember 2005 (2005-12), Seiten 1065-1076, XP002462542 ISSN: 0960-7412
- MARTÍNEZ-LÓPEZ J ET AL: "The use of fluorescent molecular beacons in real time PCR of IgH gene rearrangements for quantitative evaluation of multiple myeloma." CLINICAL AND LABORATORY HAEMATOLOGY FEB 2004, Bd. 26, Nr. 1, Februar 2004 (2004-02), Seiten 31-35, XP002462543 ISSN: 0141-9854

### SEQUENCE LISTING

<110> Identif GmbH
<120> Verfahren und Vorrichtung zum Nachweis der Authentizität einer Markierung
<130> 4645666a
<160> 4
<170> PatentIn version 3.3
<210> 1
   <211> 30
   <212> DNA
   <213> Artificial
<220>
   <223> arbitrary Sequence
<400> 1
   aagcctggag ggatgatact ttgcgcttgg 30
<210> 2
   <211> 32
   <212> DNA
   <213> Artificial
<220>
   <223> arbitrary sequence
<400> 2
   ccaagcgcaa agtatcatcc ctccaggctt gg 32
<210> 3
   <211> 27
   <212> DNA
   <213> Artificial
<220>
   <223> arbitrary sequence
<400> 3
   ccgagccacc aaaaatgata tgctcgg 27
<210> 4
   <211> 27
   <212> DNA
   <213> Artificial
<220>
   <223> arbitrary sequence
<400> 4
   ccgagccacc aaaaatgata tgctcgg 27

## Beschreibung

Die Erfindung betrifft ein Verfahren zur Authentifizierung von mit einer Markierung versehenen Gegenständen.

Die WO 01/51652 A2 offenbart ein Verfahren, bei dem ein Gegenstand zur Authentifizierung mit einer fälschungssicheren Markierung versehen wird. Die Markierung besteht aus einer ersten Nukleinsäure, welche in Form erster Flächenelemente angeordnet ist. Zur Identifizierung der Markierung wird ein durch die ersten Flächenelemente gebildetes Teilmuster sichtbar gemacht. Dazu wird die erste Nukleinsäure mit einer dazu komplementären zweiten Nukleinsäure in Kontakt gebracht. Bei einer Hybridisierung der ersten und der zweiten Nukleinsäure ist eine Fluoreszenz beobachtbar. Zur Messung eines Hintergrundsignals können neben den ersten Flächenelementen zusätzlich zweite Flächenelemente vorgesehen sein, welche eine dritte Nukleinsäure enthalten. Die dritte Nukleinsäure ist nicht komplementär zur zweiten Nukleinsäure. Zwischen der zweiten und dritten Nukleinsäure tritt keine Hybridisierung auf. Eine Messung der Fluoreszenz der zweiten Flächenelemente ermöglicht eine Bestimmung der Hintergrundfluoreszenz.

Die WO 02/072878 A1 beschreibt ein Verfahren, bei dem ein Gegenstand zur Authentifizierung mit einer Markierung versehen ist. Die Markierung umfasst ein erstes und ein zweites Flächenelement. Das erste Flächenelement ist mit einer vorgegebenen ersten und das zweite Flächenelement mit einer vorgegebenen dritten Nukleinsäure imprägniert. Zur Authentifizierung des Gegenstands werden das erste und das zweite Flächenelement mit einer Nachweislösung in Kontakt gebracht. Die Nachweislösung enthält eine zur ersten Nukleinsäure komplementäre zweite Nukleinsäure, welche mit einem Fluorophor markiert ist. Infolge einer Hybridisierung der ersten und der dazu komplementären zweiten Nukleinsäure ist ein erhöhtes Fluoreszenzsignal beobachtbar. Dagegen führt ein Kontakt der zweiten und der dritten Nukleinsäure im zweiten Flächenelement zu keiner Hybridisierung. Es ist dort keine erhöhte Fluoreszenz beobachtbar. Die Messung der Fluoreszenz im ersten und zweiten Flächenelement ermöglicht eine Identifizierung der Markierung.

In der Praxis wird als zweite Nukleinsäure meist eine Nukleinsäure mit einer Haarnadelstruktur verwendet, an deren erstem freien Ende der Fluorophor und an deren gegenüberliegendem zweiten freien Ende in einem ein Fluoreszenzsignal löschenden Abstand ein Quencher gebunden sind. Beim Inkontaktbringen der zweiten Nukleinsäure mit der ersten Nukleinsäure öffnet sich wegen der angestrebten Hybridisierung die Haarnadelstruktur und die räumliche Beziehung zwischen dem Fluorophor und dem Quencher wird aufgelöst. Infolgedessen ist das Fluoreszenzsignal beobachtbar.

Die nach dem Stand der Technik bekannten Verfahren sind in mehrfacher Hinsicht nachteilig. Zur Durchführung einer exakten Messung sind stets mit unterschiedlichen Nukleinsäuren beladene Flächenelemente in der Markierung erforderlich. Die Herstellung der Markierung ist damit aufwändig. Abgesehen davon ist es zum Nachweis der Echtheit der Markierung erforderlich, sowohl die Fluoreszenz des ersten als auch des zweiten Flächenelements zu messen und auszuwerten. Schließlich kann es durch physikalische oder chemische Prozesse auch im ersten Flächenelement zu einer unspezifischen Fluoreszenz kommen, welche zu falsch positiven oder negativen Resultaten führen kann. Beispielsweise kann das Problem auftreten, dass die angesprochene Haarnadelstruktur nicht nur durch eine Hybridisierung mit der komplementären ersten Nukleinsäure, sondern auch durch andere Einflüsse aufgehoben werden kann. Die Haarnadelstruktur kann durch eine Behandlung der zweiten Nukleinsäure mit Säuren oder Basen oder bei Überschreiten einer bestimmten Temperatur zerstört werden. Es kann also beispielsweise durch eine Imprägnierung der Markierung mit einer Säure ein falsch positives Fluoreszenzsignal erzeugt und damit die Authentizität des Gegenstands vorgetäuscht werden.

Aufgabe der Erfindung ist es, die Nachteile nach dem Stand der Technik zu beseitigen. Es soll insbesondere ein möglichst einfach durchführbares Verfahren zur Authentifizierung von mit einer Markierung versehenen Gegenständen angegeben werden, welches sich durch eine verbesserte Fälschungssicherheit und Zuverlässigkeit auszeichnet. Ferner beschrieben wird eine zur Durchführung des Verfahrens geeignete Vorrichtung .

Diese Aufgabe wird durch die Merkmale des Anspruchs 1 gelöst. Zweckmäßige Ausgestaltungen der Erfindung ergeben sich aus den Merkmalen der Ansprüche 2 bis 16.

Nach Maßgabe der Erfindung ist ein Verfahren zur Authentifizierung von mit einer Markierung versehenen Gegenständen, wobei die Markierung eine Markierungsnukleinsäure enthält, mit fol-genden Schritten vorgesehen:
a) Bereitstellen einer Referenzlösung, welche eine zumindest abschnittsweise doppelsträngige Referenznukleinsäure enthält, deren Referenznukleinsäurestränge zur Markierungsnukleinsäure nicht komplementär sind, und wobei am einen Referenznukleinsäurestrang ein erster Fluorophor und am anderen Referenznukleinsäurestrang ein erster Quencher in einem ein erstes Fluoreszenzsignal des ersten Fluorophors löschenden Abstand gebunden sind.
b) Bereitstellen einer von der Referenzlösung getrennten Nachweislösung, welche
   eine zumindest abschnittsweise doppelsträngige Nachweisnukleinsäure enthält, bei der einer der beiden Nachweisnukleinsäurestränge zur Markierungsnukleinsäure zumindest abschnittsweise komplementär ist, und wobei am einen Nachweisnukleinsäurestrang ein zweiter Fluorophor und am anderen Nachweisnukleinsäurestrang ein zweiter Quencher in einem ein zweites Fluoreszenzsignal des zweiten Fluorophors löschenden Abstand gebunden sind,
c1) Inkontaktbringen der Referenzlösung mit der Markierung unter für eine Hybridisierung einer der beiden Nachweisnukleinsäurestränge mit der Markierungsnukleinsäure geeigneten Bedingungen,
c2) Beobachten eines von der Markierung emittierten ersten Fluoreszenzsignals,
d1) Inkontaktbringen der Nachweislösung mit der Markierung unter den Bedingungen wie im Schritt lit. c1),
d2) Beobachten eines von der Markierung emittierten zweiten Fluoreszenzsignals und
   wobei die Schritte lit. d1) und lit. d2) entweder vor oder nach den Schritten lit. c1) und lit. c2) durchgeführt werden,
   und
e) Feststellen der Authentizität des Gegenstands, wenn (i) bei dem in Schritt lit. c2) beobachteten ersten Fluoreszenzsignal zumindest eine erwartete erste Eigenschaft des ersten Fluoreszenzsignals nicht beobachtbar ist, und wenn (ii) das im Schritt lit. d2) beobachtete zweite Fluoreszenzsignal zumindest einer erwarteten zweiten Eigenschaft des zweiten Fluoreszenzsignals entspricht.

Die beiden Referenznukleinsäurestränge der Referenznukleinsäure sind zur Markierungsnukleinsäure nicht komplementär. Bei Inkontaktbringen der Referenznukleinsäure mit der Markierungsnukleinsäure bleibt die doppelsträngige Struktur der Referenznukleinsäure erhalten. Ein erstes Fluoreszenzsignal ist nicht beobachtbar bzw. übersteigt nicht einen vorgegebenen Schwellwert. Bei einer Störung der Markierung, wenn z.B. Stoffe, wie Säuren, Basen oder dgl., verunreinigt oder zum Zwecke der Fälschung aufgebracht worden sind oder das Inkontaktbringen der Nachweislösung mit der Markierung unter Bedingungen, beispielsweise einer zu hohen Temperatur, erfolgt, unter denen eine doppelsträngige Struktur zweier Nukleinsäuren aufgelöst wird, ist die erwartete erste Eigenschaft des ersten Fluoreszenzsignals beobachtbar. Es ist z.B. eine Fluoreszenzintensität beobachtbar, welche den vorgegebenen Schwellwert übersteigt. Das zeigt an, dass eine falsch positive Messung vorliegt. Es kann damit auf einfache, sichere und zuverlässige Weise eine hohe Zuverlässigkeit und Fälschungssicherheit des Verfahrens gewährleistet werden.

Der eine Nachweisnukleinsäurestrang ist zumindest soweit abschnittsweise komplementär mit der Markierungsnukleinsäure, dass bei einem Inkontaktbringen der Markierungsnukleinsäure mit dem einen Nachweisnukleinsäurestrang eine Hybridisierung unter vorgegebenen Bedingungen stattfindet. Keiner der Nachweisnukleinsäurestränge ist komplementär zu den Referenznukleinsäuresträngen. Durch die Hybridisierung des Nachweisnukleinsäurestrangs mit der Markierungsnukleinsäure wird die räumliche Beziehung zwischen dem zweiten Quencher und dem zweiten Fluorophor aufgehoben. Infolgedessen kommt es bei einer Einstrahlung von Licht auf den zweiten Fluorophor zu einer beobachtbaren Fluoreszenz.

Die Markierung ist zweckmäßigerweise fest an der Oberfläche des Gegenstands angebracht. Zur Authentifizierung des Gegenstands ist es nicht erforderlich, die Markierung zu entfernen. Vorteilhafterweise kann die Markierung an Ort und Stelle, d.h. in ihrem auf der Oberfläche des Gegenstands befestigten Zustand identifiziert werden. Das macht das vorgeschlagene Verfahren besonders einfach durchführbar.

Nach dem vorgeschlagenen Verfahren wird die Markierungsnukleinsäure vorteilhafterweise zunächst mit der Referenzlösung in Kontakt gebracht und es wird das von der Markierung ggf. emittierte erste Fluoreszenzsignal beobachtet. Sofern das erste Fluoreszenzsignal einen bestimmten Schwellwert übersteigt, zeigt das an, dass die Markierung mit Störstoffen versetzt ist oder unzulässige Prüfbedingungen vorliegen. In diesem Fall ist es nicht mehr erforderlich, die Markierungsnukleinsäure nachfolgend mit der Nachweisnukleinsäure in Kontakt zu bringen. Ein besonderer Vorteil des vorgeschlagenen Verfahrens besteht darin, dass zur Beobachtung des ersten und/oder zweiten Fluoreszenzsignals ein herkömmliches Fluoreszenz-Handlesegerät bzw. eine herkömmliche Fluoreszenzmesseinrichtung verwendet werden kann, mit der insbesondere die Intensität des beobachteten Fluoreszenzsignals quantitativ erfassbar ist.

Nach einer vorteilhaften Ausgestaltung der Erfindung sind der erste Fluorophor und der erste Quencher im Bereich des einen Endes der Referenznukleinsäure gebunden. In ähnlicher Weise können der zweite Fluorophor und der zweite Quencher im Bereich des einen Endes der Nachweisnukleinsäure gebunden sein. Das Vorsehen der Fluorophor/Quencherpaare im Bereich des Endes der Referenz- und/oder Nachweisnukleinsäure ermöglicht eine besonders einfache Herstellung derselben. Die doppelsträngige Struktur wird im Falle des vorgeschlagenen endständigen Vorsehens der Fluorophor/Quencherpaare wenig gestört.

Nach einer vorteilhaften Ausgestaltung werden die Schritte lit. c1) und lit. c2) sowie die Schritte lit. d1) und lit. d2) zeitlich aufeinanderfolgend innerhalb von 120 Sekunden, vorzugsweise 60 Sekunden durchgeführt. Das ermöglicht eine Authentifizierung der Markierung vor Ort. Weiterhin kann in der Praxis sichergestellt werden, dass für die Durchführung der vorgenannten Schritte im Wesentlichen dieselben Reaktionsbedingungen vorliegen. Die Schritte lit. c1) und lit. c2) sowie die Schritte lit. d1) und lit. d2) werden zweckmäßigerweise bei Umgebungstemperatur durchgeführt. Es ist insbesondere nicht erforderlich, besondere von der Umgebungstemperatur abweichende Temperaturen einzustellen.

Nach einer zweckmäßigen Ausgestaltung liegt die Nachweisnukleinsäure in einer Haarnadelstruktur vor, bei welcher die Nachweisnukleinsäure zwei zueinander komplementäre Äste aufweist. In ähnlicher Weise kann auch die Referenznukleinsäure in einer Haarnadelstruktur vorliegen, bei welcher die Referenznukleinsäurestränge zwei zueinander komplementäre Äste aufweist. Die vorgeschlagenen selbst-komplementären Nachweis- und/oder Referenznukleinsäuren eignen sich in besonderer Weise zum Nachweis der Markierungsnukleinsäure oder ggf. falsch positiver Signale erzeugender Stoffe oder Bedingungen.

Nach einer weiteren Ausgestaltung wird beim Schritt lit. c2) und/oder d2) die Markierung mit Licht eines vorgegebenen Wellenlängenbereichs bestrahlt. Dabei umfasst der Wellenlängenbereich solche Wellenlängen, mit denen der erste und/oder zweite Fluorophor zur Erzeugung eines ersten und/oder eines zweiten Fluoreszenzsignals anregbar ist. Damit können die Intensitäten der Fluoreszenzsignale klar gegenüber dem Hintergrund erhöht und somit eine besonders sichere und zuverlässige Messung gewährleistet werden. - Zur weiteren Verbesserung der Lichtausbeute kann die Markierung auf einer Licht reflektierenden Unterlage aufgebracht sein.

Nach einer weiteren Ausgestaltung werden sowohl die Nachweisals auch die Referenzlösung auf ein einziges, die Markierungsnukleinsäure enthaltendes Nachweisfeld der Markierung aufgebracht. Indem lediglich ein einziges Nachweisfeld vorgesehen ist, kann die Erfassung der Fluoreszenz vereinfacht werden.

Vorteilhafterweise wird/werden jeweils ein vorgegebenes Volumen der Nachweis- und/oder der Referenzlösung auf die Markierung aufgetragen. Das ermöglicht eine besonders zuverlässige Authentifizierung der Markierung. Ein vorgegebenes Volumen kann mit geeigneten Flüssigkeitsapplikationsvorrichtungen aufgetragen werden. Beispielsweise können dazu eine Pipette oder ein die Nachweislösung enthaltender erster Stift sowie ein die Referenzlösung enthaltender zweiter Stift verwendet werden. Die Verwendung derartiger Flüssigkeitsapplikationsvorrichtungen vereinfacht das Handling und ermöglicht einen schnellen vor-Ort-Nachweis.

Gemessen wird jeweils zumindest eine vorgegebene erwartete Eigenschaft der ersten und zweiten Fluoreszenzsignale. Dabei kann die erwartete erste Eigenschaft der ersten Fluoreszenzsignals eine vorgegebene erste Maximalintensität in einem ersten Wellenlängenbereich und die erwartete zweite Eigenschaft eine vorgegebene zweite Mindestintensität in einem zweiten Wellenlängenbereich sein. Zur Feststellung der Authentizität des Gegenstands können der erste und der zweite Fluorophor sich auch hinsichtlich der Wellenlänge der damit erzeugten ersten und zweiten Fluoreszenzsignale unterscheiden. Zum Feststellen der Authentizität des Gegenstands kann in diesem Fall in den Wellenlängenbereichen des ersten und des zweiten Fluoreszenzsignals gemessen werden. Sofern im ersten Wellenlängenbereich ein erstes Fluoreszenzsignal mit einer oberhalb einer vorgegebenen Maximalintensität beobachtet wird, oder ein Verhältnis zwischen dem ersten und zweiten Fluoreszenzsignal bestimmt wird, welches außerhalb eines Erwartungswerts liegt, wird die Markierung als nicht authentifiziert bewertet.

Nach einer besonders vorteilhaften Ausgestaltung sind der erste und der zweite Fluorophor identisch. Auch der erste und der zweite Quencher können identisch sein. In diesem Fall kann die Anregung sowohl nach dem Auftragen der Referenzlösung als auch nach dem Auftragen der Nachweislösung mit demselben Wellenlängenbereich, d.h. mit derselben Anregungslichtquelle erfolgen. Abgesehen davon, kann auch die Beobachtung der emittierten Fluoreszenz mit ein und derselben Fluoreszenzerfassungsvorrichtung durchgeführt werden. Damit kann der technische Aufwand zur quantitativen Messung bzw. Beobachtung der Fluoreszenz vermindert werden.

Die ersten und zweiten Fluoreszenzsignale können beispielsweise mit einem Fluoreszenz-Handlesegerät erfasst werden. Dazu wird das Fluoreszenz-Handlesegerät auf die Markierung aufgesetzt, Licht zur Anregung des ersten und/oder zweiten Fluorophors erzeugt und die von der Markierung emittierte Fluoreszenz bestimmt. Es können dazu in einem jeweils vorgegebenen Wellenlängenbereich nacheinander die Intensitäten der ersten und zweiten Fluoreszenzsignale erfasst werden. Zur Bestimmung der Authentizität der Markierung können die gemessenen Intensitäten mit Erwartungswerten verglichen und bei einer Übereinstimmung mit den Erwartungswerten als authentifiziert und bei einer fehlenden Übereinstimmung mit den Erwartungswerten als nicht authentifiziert bewertet werden. Vorteilhafterweise kann es sich bei dem Erwartungswert um ein Verhältnis zwischen einer erwarteten ersten und zweiten Intensität handeln. Anstelle eines Verhältnisses kann aber auch eine Differenz oder eine andere Verknüpfung erwarteter Werte bzw. Eigenschaften mit den gemessenen Eigenschaften zum Nachweis der Intensität der Markierung verwendet werden. Die vorgenannten Erwartungswerte können anhand von Serienmessungen an intakten Markierungen und an verunreinigten Markierungen gewonnen werden.

Bei dem erfindungsgemäßen Verfahren ist es vorteilhafterweise lediglich erforderlich, die von dem einzigen Nachweisfeld emittierte Fluoreszenz zu beobachten. Es ist insbesondere nicht erforderlich, das Fluoreszenz-Handlesegerät zur Erfassung beispielsweise einer von einer gesonderten Referenzfläche emittierten Fluoreszenz über die Markierung zu bewegen. Das vereinfacht das Verfahren. - Selbstverständlich ist es auch möglich, die Markierung auf einem ersten Markierungsfeld vorzusehen und ein zweites Referenzfeld vorzusehen, welches die Markierungsnukleinsäure nicht enthält. In diesem Fall kann der Nachweis der Markierung dadurch erfolgen, dass die Nachweislösung auf das Markierungsfeld sowie auf das Referenzfeld aufgebracht wird und sowohl die vom Markierungsfeld als auch vom Referenzfeld emittierte Fluoreszenz beobachtet wird. Die vom Referenzfeld emittierte Fluoreszenz gibt die Hintergrundfluoreszenz wieder. Indem das die Hintergrundfluoreszenz wiedergebende Signal mit dem von dem Markierungsfeld emittierten Fluoreszenzsignal ins Verhältnis gesetzt wird, kann eine besonders zuverlässige Authentifizierung der Markierung erfolgen.

Vorteilhafterweise enthält die Markierung zumindest eine weitere Nukleinsäure, welche weder zu den Nachweisnukleinsäuresträngen noch zu den Referenznukleinsäuresträngen komplementär ist. Die weitere Nukleinsäure kann relativ zur Markierungsnukleinsäure im Überschuss in der Markierung enthalten sein. Die weitere Nukleinsäure dient dazu, die Markierungsnukleinsäure zu verstecken bzw. deren Isolierung aus der Markierung durch nicht autorisierte Personen unmöglich zu machen. Das erhöht weiter die Fälschungssicherheit der Markierung. Bei der weiteren Nukleinsäure kann es sich z.B. um Kalbsthymus-DNA, synthetische Oligonukleotide mit Zufallssequenzen, tRNA, Heringsperm-DNA oder Pflanzen-DNA handeln.

Nach einer weiteren Ausgestaltung der Erfindung ist vorgesehen, dass als Markierung eine die Markierungsnukleinsäure und/oder die weitere Nukleinsäure enthaltende Druckfarbe verwendet wird. Es hat sich überraschenderweise gezeigt, dass ein zuverlässiger Nachweis der Authentizität des Gegenstands auch dann erreicht werden kann, wenn die Markierungsnukleinsäure in einer Druckfarbe enthalten ist. Durch Drucken der die Markierungsnukleinsäure enthaltenden Druckfarbe kann auf besonders einfache und kostengünstige Weise eine für den Laien zunächst nicht erkennbare Markierung, beispielsweise auf Dokumenten, Geldscheinen, Tickets oder dgl., hergestellt werden.

Die Markierung kann mit einem Druckverfahren auf den zu markierenden Gegenstand oder auf ein, vorzugsweise selbstklebendes, Etikett aufgebracht werden. Das Etikett ist zweckmäßigerweise so ausgeführt, dass es nicht zerstörungsfrei vom Gegenstand gelöst werden kann.

Nach einer weiteren Ausgestaltung umfasst die Markierung eine Markierungsfläche, welche lediglich wenige Quadratmillimeter, vorzugsweise 1 bis 20 mm² groß ist. Eine solche Markierung kann kostengünstig hergestellt werden.

Zur Herstellung der Markierung werden zweckmäßigerweise 0,01 bis 1,0 pMol Markierungsnukleinsäure verwendet. Derart geringe Mengen an Markierungsnukleinsäure sind für Fälscher schwer zu isolieren, zu analysieren und nachzuahmen. Das gilt insbesondere dann, wenn die Markierungsnukleinsäure in einem Gemisch mit zumindest einer weiteren Nukleinsäure in der Markierung vorliegt.

Zum Nachweis der Markierungsnukleinsäure ist es lediglich erforderlich, wenige Mikroliter der Nachweislösung auf die Markierung aufzubringen. Vorteilhafterweise werden definierte vorgegebene Volumina der Referenz- und Nachweislösung auf die Markierung aufgetragen. Dadurch wird gewährleistet, dass die erwarteten ersten und zweiten Fluoreszenzsignäle sich in einem engen Intensitätsbereich befinden. Es ist insbesondere nicht erforderlich, die Markierung in der Nachweisflüssigkeit unterzutauchen oder die Markierung nach dem Aufbringen der Nachweisflüssigkeit zu waschen, um zum Feststellen der Authentizität eine geeignete Fluoreszenzemission zu beobachten. Damit ist es insbesondere möglich, den Gegenstand mit der darauf angebrachten Markierung zu authentifizieren. Durch das Aufbringen der geringen Mengen an Nachweisflüssigkeit wird der Gegenstand in keiner Weise beeinflusst. Die Authentizität des Gegenstands kann also schnell und einfach festgestellt werden.

Außerdem wird hier beschrieben ein Kit mit einer die Referenzlösung enthaltenden ersten Flüssigkeitsspendevorrichtung vorgesehen, wobei die Referenzlösung eine zumindest abschnittsweise doppelsträngige Referenznukleinsäure enthält, deren Referenznukleinsäurestränge zu einer vorgegebenen Markierungsnukleinsäure nicht komplementär sind, und wobei am einen Referenznukleinsäurestrang ein erster Fluorophor und am anderen Referenznukleinsäurestrang ein erster Quencher in einem ein erstes Fluoreszenzsignal des ersten Fluorophors löschenden Abstand gebunden sind, und einer die Nachweislösung enthaltenden zweiten Flüssigkeitsspendeeinrichtung, wobei die Nachweislösung eine zumindest abschnittsweise doppelsträngige Nachweisnukleinsäure enthält, bei der einer der beiden Nachweisnukleinsäurestränge zur vorgegebenen Markierungsnukleinsäure zumindest abschnittsweise komplementär ist, und wobei am einen Nachweisnukleinsäurestrang ein zweiter Fluorophor und am anderen Nachweisnukleinsäurestrang ein zweiter Quencher in einem ein zweites Fluoreszenzsignal des zweiten Fluorophors löschenden Abstand gebunden sind.

Vorteilhafterweise sind die erste und die zweite Flüssigkeitsspendeeinrichtung jeweils ein die Nachweis- oder Referenzlösung enthaltender Stift oder eine Pipette.

Es kann anstelle des Kits auch eine Flüssigkeitsspendeeinrichtung mit einem ersten Behälter zur Aufnahme der Referenzlösung und einem zweiten Behälter zur Aufnahme der Nachweislösung und eine Einrichtung zur zeitlich aufeinanderfolgenden separaten Abgabe eines vorgegebenen Volumens der Referenz- und der Nachweislösung vorgesehen sein. Bei der Flüssigkeitsspendeeinrichtung kann es sich um eine Doppelpipette handeln, welche mit einem geeigneten Mechanismus versehen ist, bei dessen Betätigung zunächst ein vorgegebenes Volumen an Referenzlösung und bei dessen erneuter Betätigung nachfolgend ein vorgegebenes Volumen an Nachweislösung abgegeben wird. Das vereinfacht die Handhabung des Nachweises. Eine Verwechslung der Reihenfolge des Auftragens der Referenz- und der Nachweislösung ist damit geräteseitig ausgeschlossen.

Bei dem markierten Gegenstand handelt es sich bevorzugt um Güter, Waren, Verpackungen und Dokumente. Insbesondere kann es sich dabei um Markenprodukte, Zahlungsmittel, Chipkarten oder Verpackungen dafür handeln. Der zu authentifizierende Gegenstand wird vom Hersteller in den freien Warenverkehr gegeben und unterliegt damit einer potenziellen Fälschungsgefahr. Die Authentizität des Gegenstands kann bei Bedarf unter Anwendung des erfindungsgemäßen Verfahrens sicher und zuverlässig bestimmt werden. Dazu ist es lediglich erforderlich, die beispielsweise in einem Filzstift aufgenommene Nachweislösung auf die Markierung aufzutragen und anschließend mit einem Fluoreszenz-Handlesegerät die von der Markierung emittierte Fluoreszenz zu beobachten, zu analysieren bzw. mit vorgegebenen Erwartungswerten zu vergleichen und infolge des Ergebnisses des Vergleichs die Authentizität der Markierung festzustellen.

Nachfolgend werden Ausführungsbeispiele der Erfindung anhand der Zeichnungen näher erläutert. Es zeigen:
- Fig. 1: schematisch eine Markierungs-, Nachweis- und eine Referenznukleinsäure,
- Fig. 2a bis 2c: schematisch Reaktionsvarianten beim Kontakt einer Markierungsnukleinsäure mit einer Re- ferenz- und Nachweisnukleinsäure und
- Fig. 3a bis 3e: schematisch aufeinander folgende Verfah- rensschritte.

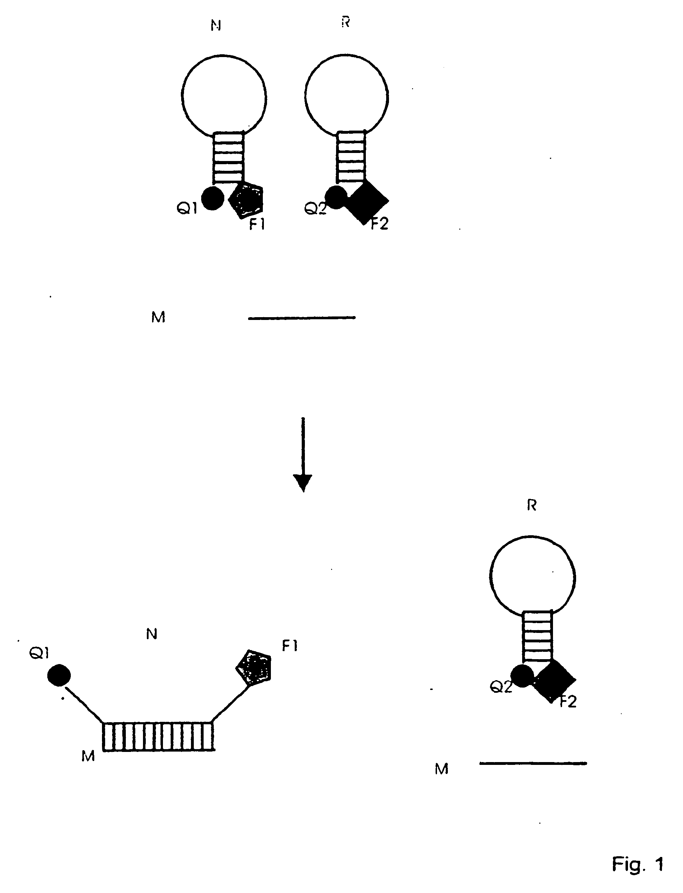

Fig. 1 zeigt schematisch eine Markierungs- M, eine Nachweis- N und eine Referenznukleinsäure R. Die Nachweisnukleinsäure N und die Referenznukleinsäure R sind jeweils nach Art eines Molecular Beacon ausgebildet. Die Nachweisnukleinsäure N umfasst eine Haarnadelstruktur, an deren freien Enden ein erstes Fluorophor F1 und ein erster Quencher Q1 in einem die Fluoreszenz des ersten Fluorophors F1 löschenden Abstand gebunden sind. Desgleichen umfasst die Referenznukleinsäure R eine Haarnadelstruktur, an deren freien Enden ein zweiter Fluorophor F2 und ein zweiter Quencher Q2 in einem die Fluoreszenz des zweiten Fluorophors F2 löschenden Abstand gebunden sind.

Die Nachweisnukleinsäure N ist zumindest abschnittsweise komplementär zur Markierungsnukleinsäure M. Beim Inkontaktbringen der Nachweisnukleinsäure N mit der Markierungsnukleinsäure M kommt es zur Hybridisierung. Der die Fluoreszenz des ersten Fluorophors F1 löschende Abstand im ersten Quencher Q1 - wird aufgehoben. Bei einer Anregung des ersten Fluorophors F1 ist eine Fluoreszenz beobachtbar. Die Referenznukleinsäure R ist dagegen nicht komplementär zur Markierungsnukleinsäure M ausgebildet. Beim Inkontaktbringen der Referenznukleinsäure R mit der Markierungsnukleinsäure M kommt es nicht zu einer Hybridisierung. Infolgedessen bleibt die räumliche Beziehung zwischen dem zweiten Quencher Q2 und dem zweiten Fluorophor F2 erhalten. Bei einer Anregung des zweiten Fluorophors F2 kommt es nicht zur Ausbildung einer Fluoreszenz.

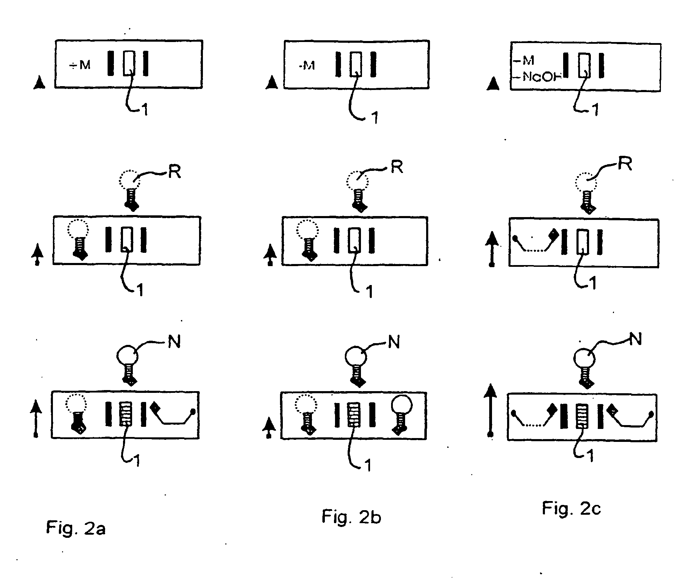

Die in Fig. 2a untereinander dargestellten Abbildungen zeigen schematisch eine erste Reaktionsvariante, bei welcher eine Markierung 1 eine Markierungsnukleinsäure M enthält. Die Markierung 1 ist in diesem Fall frei von Störstoffen. Sie befindet sich auf Umgebungstemperatur, d.h. auf einer Temperatur von 20°C ± 5°C. Die Markierungsnukleinsäure M wird in einem ersten Schritt mit der Referenzlösung in Kontakt gebracht. Da die Markierung keine Störstoffe enthält und keine unzulässig hohen Temperaturen ausgesetzt ist, bleibt die ursprüngliche Struktur einer Referenznukleinsäure R erhalten. Es ist kein oder nur ein schwaches zweites Fluoreszenzsignal beobachtbar. In einem zweiten Schritt wird nachfolgend eine eine Nachweisnukleinsäure N enthaltende Nachweislösung auf die Markierung 1 aufgetragen. Die Nachweisnukleinsäure N hybridisiert mit der Markierungsnukleinsäure M. Die räumliche Beziehung zwischen dem ersten Fluorophor und dem ersten Quencher wird aufgehoben. Es ist ein erstes Fluoreszenzsignal beobachtbar, anhand dessen die Authentizität der Markierung 1 erkannt werden kann.

Bei der in Fig. 2b gezeigten zweiten Reaktionsvariante enthält die Markierung 1 keine Markierungsnukleinsäure M. Sie ist frei von Störstoffen und befindet sich auf Umgebungstemperatur. In diesem Fall wird infolge einer fehlenden Hybridisierung mit der Nachweisnukleinsäure N die räumliche Struktur der Nachweisnukleinsäure N nicht geändert. Es ist in diesem Fall lediglich ein schwaches Fluoreszenzsignal beobachtbar. Die Markierung wird in diesem Fall als nicht authentisch angesehen.

Fig. 2c zeigt eine dritte Reaktionsvariante. Dabei enthält die Markierung 1 die Markierungsnukleinsäure M. Ferner enthält die Markierung 1 Störstoffe, beispielsweise NaOH. Beim Inkontaktbringen der Markierung 1 mit der Referenznukleinsäure R wird die Haarnadelstruktur der Referenznukleinsäure R infolge des herrschenden pH-Werts zerstört. Es ist in diesem Fall ein deutliches Fluoreszenzsignal beobachtbar. Dieses bereits im ersten Verfahrensschritt beobachtbare Fluoreszenzsignal zeigt an, dass die Authentizität der Markierung nicht nachweisbar ist. Sofern in einem zweiten Verfahrensschritt zusätzlich die Nachweislösung mit der Markierung in Kontakt gebracht wird, hybridisiert die Nachweisnukleinsäure N mit der Markierungsnukleinsäure M. Es wird eine weitere Erhöhung des Fluoreszenzsignals beobachtet. Das beobachtbare Fluoreszenzsignal setzt sich in diesem Fall sowohl aus dem von der Referenznukleinsäure R als auch aus dem von der Nachweisnukleinsäure N erzeugten Fluoreszenzsignalen zusammen. Die Intensität der Fluoreszenzsignale ist in diesem Fall so stark, dass sie einen vorgegebenen Grenzwert überschreitet. In diesem Fall wird die Markierung 1 wiederum als nicht authentisch angesehen.

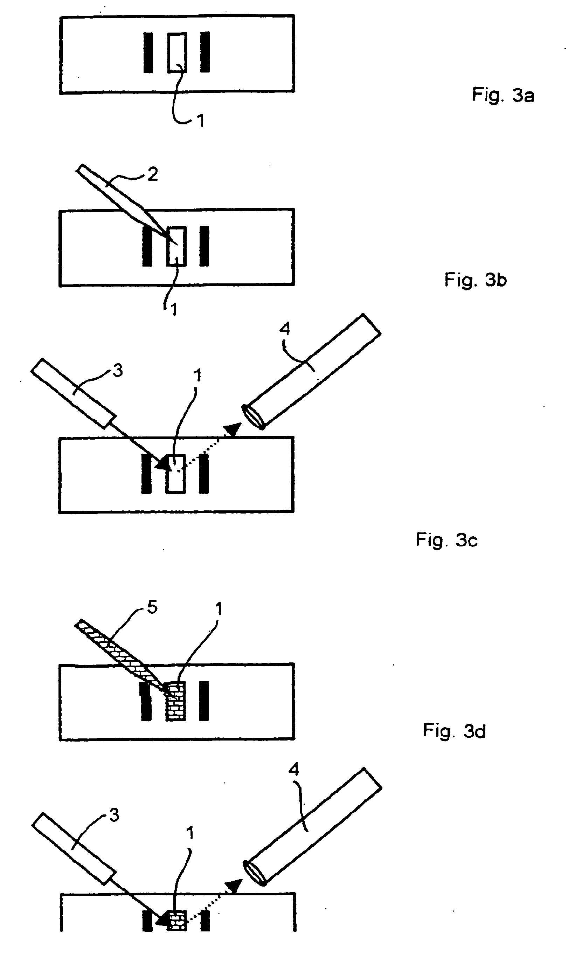

In den Fig. 3a bis 3e sind nochmals schematisch die Schritte des erfindungsgemäßen Verfahrens dargestellt. Fig. 3a zeigt die erfindungsgemäße Markierung 1 mit einem einzigen Markierungsfeld. Das Markierungsfeld besteht im Wesentlichen aus einer aufgedruckten Druckfarbe, welche Markierungsnukleinsäure M enthält. Bei dem in Fig. 3b gezeigten ersten Verfahrensschritt wird unter Verwendung eines ersten Stifts 2 Referenzlösung auf das Markierungsfeld aufgetragen. Anschließend wird mit einer Anregungslichtquelle 3 das Markierungsfeld beleuchtet und die damit ggf. angeregte Fluoreszenz mittels einer Fluoreszenzmesseinrichtung 4 beobachtet. Mit der Fluoreszenzmesseinrichtung 4 ist es insbesondere möglich, eine Intensität der von der Markierungsfläche abgestrahlten Fluoreszenz quantitativ zu bestimmen.

In einem nachfolgenden weiteren Verfahrensschritt wird sodann unter Verwendung eines zweiten Stifts 5 Nachweislösung auf das einzige Markierungsfeld aufgetragen. Wie in Fig. 3e gezeigt ist, wird nachfolgend wiederum unter Verwendung der Anregungslichtquelle 3 sowie der Fluoreszenzmesseinrichtung 4 die vom Markierungsfeld emittierte Fluoreszenz quantitativ erfasst.

Die Flucreszenzmesseinrichtung 4 kann mit einer geeigneten Auswertevorrichtung versehen sein, in der Schwellwerte und Grenzwerte hinterlegt sind. Damit kann automatisch anhand der gemessenen Fluoreszenzintensitäten ermittelt werden, ob die geprüfte Markierung authentisch ist oder nicht.

### Beispiel 1:

### Herstellung der Markierung

Tn einer herkömmlichen Druckerfarbe (UV-Tinte der Firma Wolke Inks und Printers GmbH, Hersbruck, Deutschland, Art.-Nr. 690900) ist als Markierungsnukleinsäure eine Nukleinsäure gemäß Sequenzprotokoll Nr. 1 (5'-AAGCCTGGAGGGATGATACTTTGCGCTTGG-3') in einer Konzentration von 1 mg/ml aufgenommen. Die Markierungsnukleinsäure wurde mittels Standard-Amidit-Festphasensynthese hergestellt.

Die Druckfarbe ist z.B. im Inkjet-Druckverfahren auf einen Träger, z.B weißes Papier, eine geeignete Kunststofffolie oder dgl., mit einem Drucker, z.B. mittels des Druckers m600 der Firma Wolke Inks und Printers GmbH, Hersbruck, Deutschland, auf eine Fläche von 4 mm x 3 mm aufgedruckt. Aus einem solchermaßen bedruckten Träger kann ein, vorzugsweise selbstklebendes, Etikett hergestellt werden.

Es wurden Kontrollmarkierungen ohne Markierungsnukleinsäure durch Verdrucken der Druckerfarbe ohne Zusatz von Markierungsnukleinsäure hergestellt.

### Nachweis der Markierung

Zur Authentifizierung des mit der Markierung markierten Gegenstands wurde auf zehn Markierungen eine Referenzlösung aufgetragen. Die Referenzlösung war zu diesem Zweck in einem ersten Stift aufgenommen, der bei Kontakt mit der Markierung Referenzlösung auf die Markierung überträgt. Bei der Referenzlösung handelt es sich um eine wässrige Flüssigkeit, in der als Referenznukleinsäure eine Nukleinsäure gemäß Sequenzprotokoll Nr. 3 (5'-FAM-CCG AGC CAC CAA AAA TGA TAT GCT CGG-3'-DABCYL) in einer Konzentration von je 1 µMol/l in TEN (10 mM TrisCl, pH 8; 1 mM EDTA, 100 mM NaCl, 0,19% SDS (w/v)) enthalten ist. Nach Aufnahme eines ersten Fluoreszenzsignals erfolgte die Aufgabe der Nachweislösung auf die Markierung. Bei der Nachweislösung handelt es sich um eine wässrige Flüssigkeit, in der als Nachweisnukleinsäure eine Nukleinsäure gemäß Sequenzprotokoll Nr. 2,5-FAM-CCAAGCGCAAAGTATCATCCCTCCAGGCTTGG-DABCYL- enthalten ist. Diese ist teilweise komplementär zu der in der Markierung enthaltenen Markierungsnukleinsäure gemäß Sequenzprotokoll Nr. 1. Die Nachweisnukleinsäure lag ebenfalls in einer Konzentration von 1 µMol/l in TEN (10 mM-2-Amino-2-(hydroxylmethyl)-1,3-propanediol (TRIS), pH 8; 1 mM EDTA, 100 mM NaCl, 0,1% SDS (w/v)) vor. Nach dem Aufbringen der Nachweislösung ist das ggf. von der Nachweisnukleinsäuresequenz erzeugte zweite Fluoreszenzsignal gemessen bzw. beobachtet worden.

An den freien Enden der Nachweisnukleinsäure ist ein zweites Fluorophor/Quencherpaar in einem Abstand gebunden, welcher ein vom zweiten Fluorophor erzeugtes Fluoreszenzsignal löscht. Das zweite Fluorophor kann beispielsweise eine Fluoreszenzgruppe oder ein Derivat davon sein; der zweite Quencher kann beispielsweise Dabcyl oder ein Blackhole-Quencher (Jena Bioscience GmbH, Loebstedter Strasse 80, D-07749 Jena, Germany) sein. Weitere geeignete Kombinationen für Fluorophor/Quencherpaare sind z.B. in Tyagi S, Bratu DP, and Kramer FR (1998) "Multicolor molecular beacons for allele discrimination"; Nat Biotechnol 16, 49-53 offenbart. - Die Referenznukleinsäure kann mit demselben Fluorophor/Quencherpaar wie die Nachweisnukleinsäure versehen sein.

Die Nachweis- und Referenznukleinsäuren wurden mittels Standard-Amidit-Festphasensynthese hergestellt. Die Markierungsnukleinsäure ist im vorliegenden Beispiel terminal selbstkomplementär ausgebildet und liegt in einer Haarnadelstruktur vor.

Beim Inkontaktbringen der Referenzlösung mit der Markierungsnukleinsäure kommt es zu keiner Hybridisierung zwischen der Referenznukleinsäure und der Markierungsnukleinsäure. Infolge dessen wird die doppelsträngige Struktur der Referenznukleinsäure nicht aufgelöst und damit die ursprüngliche räumliche Beziehung zwischen dem ersten Fluorophor und dem ersten Quencher nicht aufgehoben. Bei einer Anregung mit Licht einer Wellenlänge mit 492 nm ist ein geringes Fluoreszenzsignal bei 517 nm beobachtbar, welches insbesondere durch die Eigenfluoreszenz der Markierungsnukleinsäure hervorgerufen wird. Die Messung erfolgt bevorzugt wenige Sekunden bis Minuten nach dem Kontakt mit der Referenzlösung. Die Messung kann mit Hilfe eines Fluoreszenz-Handlesegeräts durchgeführt werden. Das ermöglicht einen Nachweis auf dem markierten Objekt. Fluoreszenz-Handlesegerät mit den geforderten Eigenschaften können bei der identif GmbH, Erlangen erworben werden.

Beim Inkontaktbringen der Nachweislösung mit der Markierungsnukleinsäure kommt es zu einer Hybridisierung zwischen der Nachweisnukleinsäure und der Markierungsnukleinsäure. Infolgedessen wird die Struktur der Nachweisnukleinsäure verändert und damit die ursprüngliche räumliche Beziehung zwischen dem zweiten Fluorophor und dem zweiten Quencher aufgehoben. Bei einer Anregung mit Licht einer Wellenlänge mit 492 nm ist ein Fluoreszenzsignal bei 517 nm beobachtbar, welches durch die Fluoreszenz der Markierungsnukleinsäure hervorgerufen wird. Die Messung erfolgt bevorzugt wenige Sekunden bis Minuten nach dem Kontakt mit der Nachweislösung. Die Messung kann mit Hilfe eines Fluoreszenz-Handlesegeräts durchgeführt werden. Geeignete Fluoreszenz-Handlesegerät können bei der Firma identif GmbH, Erlangen erworben werden.

**Tabelle: 1**

| Markierung | Signale + Markierungsnukleinsäure | | Signale Markierungsnukleinsäure | | Signale -Markierungsnukleinsäure + NaOH | |
|---|---|---|---|---|---|---|
| | Nachweislösung | Nachweislösung | Referenzlösung | Nachweislösung | Referenzlösung | Nachweislösung |
| 1 | 287 | 531 | 266 | 291 | 481 | 722 |
| 2 | 293 | 540 | 257 | 289 | 472 | 699 |
| 3 | 291 | 562 | 283 | 314 | 478 | 711 |
| 4 | 278 | 545 | 280 | 317 | 495 | 725 |
| 5 | 266 | 543 | 294 | 323 | 473 | 707 |
| 6 | 285 | 563 | 289 | 327 | 481 | 720 |
| 7 | 274 | 539 | 263 | 312 | 460 | 695 |
| 8 | 293 | 543 | 282 | 317 | 492 | 723 |
| 9 | 275 | 548 | 277 | 308 | 475 | 701 |
| 10 | 284 | 560 | 283 | 321 | 471 | 697 |
| Mittelwert | 283 | 548 | 278 | 312 | 488 | 710 |
| Erwartungswert | < 350 | > 350 | < 350 | < 350 | > 350 | |

Tabelle 1 zeigt die Auswertung von zehn Markierungen nach der Auftragung der Referenz- und Nachweisnukleinsäuren. In den Spalten ist der Wert für die Fluoreszenz der Nachweisnukleinsäure bei 517 nm angegeben. Es wurden Markierungen mit und ohne Markierungsnukleinsäure verwendet. Ferner wurden Markierungen ohne Markierungsnukleinsäure verwendet, welche mit Störstoffen (Markierungsnukleinsäure + NaOH) versetzt waren.

Der Wert für die Referenznukleinsäure liegt in einem engen Bereich um den Mittelwert von 283. Dieser Wert entspricht der geschlossenen Struktur der Referenznukleinsäure, in der erster Fluorophor und erster Quencher durch die Hybridisierung mit der Nachweisnukleinsäure räumlich eng benachbart vorliegen (Spalte links: Signale + Markierungsnukleinsäure). - Der Wert für die Nachweisnukleinsäure liegt in einem engen Bereich um den Mittelwert von 548. Dieser Wert entspricht der offenen Struktur der Nachweisnukleinsäure, in der zweiter Fluorophor und zweiter Quencher durch die Hybridisierung mit der komplementären Markierungsnukleinsäure räumlich getrennt vorliegen. Der vorgenannte Wert enthält außerdem das durch die geschlossene Struktur der Referenznukleinsäure erzeugte Signal (Spalte rechts: Signale + Markierungsnukleinsäure).

In den Spalten Signale + Markierungsnukleinsäure wurden gedruckte Markierungen ohne Markierungsnukleinsäure verwendet. Der Wert nach Aufbringen der Referenzlösung liegt in einem Bereich um einen Mittelwert von 278. Dieser Wert entspricht der geschlossenen Struktur der Referenznukleinsäure, in der erster Fluorophor und erster Quencher bei Abwesenheit einer Markierungsnukleinsäure durch die Rückfaltung räumlich nah benachbart vorliegen. Nach dem Aufbringen der Nachweislösung liegt der Wert in einem engen Bereich um den Mittelwert von 312. Dieser Wert entspricht der geschlossenen Struktur sowohl der Referenzals auch der Nachweisnukleinsäuren.

In den Spalten Markierungsnukleinsäure + NaOH wurden gedruckte Markierungen ohne Markierungsnukleinsäure verwendet, denen vor dem Kontakt mit der Referenz- und Nachweisflüssigkeit 0,5 µl einer 1 molaren NaOH-Lösung zugefügt wurden. Das Zufügen soll hier eine mögliche Störung der Markierung simulieren. Der Wert für die Referenznukleinsäure liegt im Bereich um einen Mittelwert von 488. Dieser Wert entspricht der geöffneten Struktur der Referenznukleinsäure, bei welcher der erste Fluorophor und der erste Quencher durch die Denaturierung wegen des alkalischen pHs räumlich getrennt vorliegen. Der Wert nach dem Auftragen der Nachweislösung schwankt um einen Mittelwert von 710. Dieser Wert entspricht der Summe der Signale aus der geöffneten Struktur der Referenznukleinsäure sowie der Nachweisnukleinsäure. Durch die denaturierende Wirkung des alkalischen pH liegt auch die Nachweisnukleinsäure in der geöffneten Struktur vor.

Die deutliche Trennung der Fluoreszenz-Werte bei An- und Abwesenheit der Markierungsnukleinsäure ermöglicht eine Festlegung von Erwartungswerten zur Beurteilung der Authentizität der Markierung. Beispielsweise kann für die Bewertung der Markierung ein Erwartungswert von über 350 für die Nachweisnukleinsäure und ein Erwartungswert von unter 350 für die Referenznukleinsäure festgelegt werden. Beide Kriterien müssen für eine Bewertung der Markierung als authentisch erfüllt werden. Nach diesen Kriterien sind alle Markierungen, welche Markierungsnukleinsäure enthalten (Tabelle 1, Signale + Markierungsnukleinsäure) als authentisch bewertet, da sämtliche Werte für die Nachweisnukleinsäure oberhalb von 350 liegen und sämtliche Werte für die Referenznukleinsäure unterhalb von 350 liegen.

Ein Fehlen der Markierungsnukleinsäure (Tabelle 1, Spalten Signale - Markierungsnukleinsäure) führt zu Werten unterhalb von 350 für die Referenz- und Nachweisnukleinsäure. Damit liegt der Wert die Nachweisnukleinsäure unterhalb des Erwartungswertes. Die Kriterien für die Authentizität der Markierung sind nicht erfüllt.

Bei Anwesenheit von Störstoffen in der Markierung erhöht sich der Wert der Fluoreszenz der Referenznukleinsäure auf Werte oberhalb des Erwartungswerts für die Referenznukleinsäure (Tabelle 1, Spalte - Markierungsnukleinsäure + NaOH). Das Überschreiten des Erwartungswerts für die Referenznukleinsäure führt ebenfalls zu einer Bewertung der Markierung als nicht authentisch.

## Patentansprüche

1. Verfahren zur Authentifizierung von mit einer Markierung versehenen Gegenständen, wobei die Markierung eine Markierungsnukleinsäure enthält, mit folgenden Schritten:
a) Bereitstellen einer Referenzlösung, welche eine zumindest abschnittsweise doppelsträngige Referenznukleinsäure enthält, deren Referenznukleinsäurestränge zur Markierungsnukleinsäure nicht komplementär sind, und wobei am einen Referenznukleinsäurestrang ein erster Fluorophor und am anderem Referenznukleinsäurestrang ein erster Quencher in einem ein erstes Fluoreszenzsignal des ersten Fluorophors löschenden Abstand gebunden sind,
b) Bereitstellen einer von der Referenzlösung getrennten Nachweislösung, welche
eine zumindest abschnittsweise doppelsträngige Nachweisnukleinsäure enthält, bei der einer der beiden Nachweisnukleinsäurestränge zur Markierungsnukleinsäure zumindest abschnittsweise komplementär ist, und wobei am einen Nachweisnukleinsäurestrang ein zweiter Fluorophor und am anderen Nachweisnukleinsäurestrang ein zweiter Quencher in einem ein zweites Fluoreszenzsignal des zweiten Fluorophors löschenden Abstand gebunden sind,
c1) Inkontäktbringen der Referenzlösung mit der Markierung unter für eine Hybridisierung einer der beiden Nachweisnukleinsäurestränge mit der Markierungsnukleinsäure geeigneten Bedingingen,
c2) Beobachten eines von der Markierung emittierten ersten Fluoreszenzsignals,
d1) Inkontaktbringen der Nachweislösung mit der Markierung unter den Bedingungen wie im Schritt lit. c1),
d2) Beobachten eines von der Markierung emittierten zweiten Fluoreszenzsignals und
wobei die Schritte lit. d1) und lit. d2) entweder vor oder nach den Schritten lit. c1) und lit. c2) durchgeführt werden,
und
e) Feststellen der Authentizität des Gegenstands, wenn (i) bei dem im Schritt lit. c2) beobachteten ersten Fluoreszenzsignal zumindest eine erwartete erste Eigenschaft des ersten Fluoreszenzsignals nicht beobachtbar ist und wenn (ii) das im Schritt lit. d2) beobachtete zweite Fluoreszenzsignal zumindest einer erwarteten zweiten Eigenschaft des zweiten Fluoreszenzsignals entspricht.

2. Verfahren nach Anspruch 1, wobei der erste Fluorophor und der erste Quencher im Bereich des einen Endes der Referenznukleinsäure gebunden sind.

3. Verfahren nach einem der vorhergehenden Ansprüche, wobei der zweite Fluorophor und der zweite Quencher im Bereich des einen Endes der Nachweisnukleinsäure gebunden sind.

4. Verfahren nach einem der vorhergehenden Ansprüche, wobei die Schritte lit. c1) und lit. c2) sowie die Schritte lit. d1) und lit. d2) zeitlich aufeinander folgend innerhalb von 120 Sekunden, vorzugsweise innerhalb von 60 Sekunden durchgeführt werden.

5. Verfahren nach einem der vorhergehenden Ansprüche, wobei die Schritte lit. c1) und lit. c2) sowie die Schritte lit.
d1) und lit. d2) bei Umgebungstemperatur durchgeführt werden.

6. Verfahren nach einem der vorhergehenden Ansprüche, wobei die Nachweisnukleinsäure in einer Haarnadelstruktur vorliegt, bei welcher die Nachweisnukleinsäure zwei zueinander komplementäre Äste aufweist.

7. Verfahren nach einem der vorhergehenden Ansprüche, wobei die Referenznukleinsäure in einer Haarnadelstruktur vorliegt, bei welcher die Referenznukleinsäurestränge zwei zueinander komplementäre Äste aufweist.

8. Verfahren nach einem der vorhergehenden Ansprüche, wobei beim Schritt lit. c2) und/oder lit. d2) die Markierung mit Licht eines vorgegeben Wellenlängenbereichs bestrahlt wird.

9. Verfahren nach einem der vorhergehenden Ansprüche, wobei die Markierung auf einer Licht reflektierenden Unterlage aufgebracht ist.

10. Verfahren nach einem der vorhergehenden Ansprüche, wobei sowohl die Nachweis- als auch die Referenzlösung auf ein einziges, die Markierungsnukleinsäure enthaltendes Nachweisfeld der Markierung aufgebracht werden.

11. Verfahren nach einem der vorhergehenden Ansprüche, wobei jeweils ein vorgegebenes Volumen der Nachweis- und/oder die Referenzlösung auf die Markierung aufgetragen wird/werden.

12. Verfahren nach einem der vorhergehenden Ansprüche, wobei die erwartete erste Eigenschaft eine vorgegebene Maximalintensität in einem ersten Wellenlängenbereich und die erwartete zweite Eigenschaft eine vorgegebene Mindestintensität in einem zweiten Wellenlängenbereich ist.

13. Verfahren nach einem der vorhergehenden Ansprüche, wobei der erste und zweite Fluorophor identisch sind.

14. Verfahren nach einem der vorhergehenden Ansprüche, wobei die Markierung außer der Markierungsnukleinsäure zumindest eine weitere Nukleinsäure enthält.

15. Verfahren nach einem der vorhergehenden Ansprüche, wobei als Markierung eine die Markierungsnukleinsäure und/oder die weitere Nukleinsäure enthaltende Druckfarbe verwendet wird.

16. Verfahren nach einem der vorhergehenden Ansprüche, wobei die Markierung mit einem Druckverfahren auf den zu markierenden Gegenstand der auf ein, vorzugsweise selbstklebendes, Etikett aufgebracht wird.

## Claims

1. A method of authenticating objects which are provided with a mark, said mark comprising a mark nucleic acid, wherein method comprises:
a) providing a reference solution, which comprises a reference nucleic acid that is double-stranded at least in sections, wherein the reference nucleic acid strands of the reference nucleic acid are not complementary to the mark nucleic acid, and wherein a first fluorophore is bound to one reference nucleic acid strand and a first quencher is bound to the other reference nucleic acid strand at a distance that quenches a first fluorescence signal of the first fluorophore,
b) providing a detection solution that is separate from the reference solution, which comprises a detection nucleic acid that is double-stranded at least in sections, wherein one of the two detection nucleic acid strands is complementary to the mark nucleic acid at least in sections, and wherein a second fluorophore is bound to one detection nucleic acid strand and wherein a second quencher is bound to the other detection nucleic acid strand at a distance that quenches a second fluorescence signal of the second fluorophore,
c1) contacting the reference solution with the mark under conditions suitable for hybridization of one of the two detection nucleic acid strands with the mark nucleic acid,
c2) observing a first fluorescence signal emitted by the mark,
d1) contacting the detection solution with the mark under the conditions as in step c1),
d2) observing a second fluorescence signal emitted by the mark and
wherein steps d1) and d2) are carried out either before or after steps c1) and c2), and
e) establishing authenticity of the object, if (i) for the first fluorescence signal observed in step c2) at least one expected first property of the first fluorescence signal is not observable, and if (ii) the second fluorescence signal observed in step d2) corresponds to at least one expected second property of the second fluorescence signal.

2. Method of claim 1, wherein the first fluorophore and the first quencher are bound in the region of one end of the reference nucleic acid.

3. Method of the preceding claims, wherein the second fluorophore and the second quencher are bound in the region of one end of the detection nucleic acid.

4. Method of the preceding claims, wherein steps c1) and c2) and steps d1) and d2) are carried out sequentially within 120 seconds, preferably within 60 seconds.

5. Method of any of the preceding claims, wherein steps c1) and c2) and steps d1) and d2) are carried out at ambient temperature.

6. Method of any of the preceding claims, wherein the detection nucleic acid is present in a hairpin structure, in which the detection nucleic acid has two mutually complementary branches.

7. Method of any of the preceding claims, wherein the reference nucleic acid is present in a hairpin structure, in which the reference nucleic acid strands have two mutually complementary branches.

8. Method of any of the preceding claims, wherein in step c2) and/or d2) the mark is irradiated with light of a predefined wavelength range.

9. Method of any of the preceding claims, wherein the mark is applied on a light-reflecting substrate.

10. Method of any of the preceding claims, wherein both the detection solution and the reference solution are applied on a single detection field of the mark which contains the mark nucleic acid.

11. Method of any of the preceding claims, wherein in each case a predefined volume of the detection and/or the reference solution is applied on the mark.

12. Method of any of the preceding claims, wherein the expected first property is a predefined maximum intensity in a first wavelength range and the expected second property is a predefined minimum intensity in a second wavelength range.

13. Method of any of the preceding claims, wherein the first and second fluorophores are identical.

14. Method of any of the preceding claims, wherein the mark contains, apart from the mark nucleic acid, at least one additional nucleic acid.

15. Method of any of the preceding claims, wherein a printing ink containing the mark nucleic acid and/or the additional nucleic acid is used as the mark.

16. Method of any of the preceding claims, wherein the mark is applied by a printing process on the object to be marked or on a, preferably self-adhesive, label.

## Revendications

1. Procédé d'authentification d'objets pourvus d'un marquage, dans lequel le marquage contient un acide nucléique de marquage, comprenant les étapes consistant à :
a) préparer une solution de référence qui contient un acide nucléique de référence, au moins par segments, à double brin, les brins d'acide nucléique de référence n'étant pas complémentaires de l'acide nucléique de marquage et dans lequel un premier fluorophore sur un premier brin d'acide nucléique de référence et un premier agent d'extinction sur l'autre brin d'acide nucléique de référence sont liés à une distance étouffant un premier signal de fluorescence du premier fluorophore,
b) préparer une solution indicatrice séparée de la solution de référence, qui contient un acide nucléique indicateur, au moins par segments, à double brin, l'un des deux brins d'acide nucléique indicateur étant complémentaire, au moins par segments, de l'acide nucléique de marquage, et dans lequel un deuxième fluorophore sur un brin d'acide nucléique indicateur et un deuxième agent d'extinction sur l'autre brin d'acide nucléique indicateur sont liés à une distance étouffant un deuxième signal de fluorescence du deuxième fluorophore,
c1) mettre en contact la solution de référence avec le marquage dans des conditions appropriées pour une hybridation d'un des deux brins d'acide nucléique indicateur avec l'acide nucléique de marquage,
c2) observer un premier signal de fluorescence émis par le marquage,
d1) mettre en contact la solution indicatrice avec le marquage dans les conditions indiquées à l'étape c1),
d2) observer un deuxième signal de fluorescence émis par le marquage, et
dans lequel les étapes d1) et d2) sont réalisées avant ou après les étapes c1) et c2),
et
e) établir l'authenticité de l'objet, si (i), dans le premier signal de fluorescence observé à l'étape c2), au moins une première propriété escomptée du premier signal de fluorescence n'est pas observable et si (ii) le deuxième signal de fluorescence observé à l'étape d2) correspond à au moins une deuxième propriété escomptée du deuxième signal de fluorescence.

2. Procédé selon la revendication 1, dans lequel le premier fluorophore et le premier agent d'extinction sont liés dans la zone d'une des extrémités de l'acide nucléique de référence.

3. Procédé selon l'une quelconque des revendications précédentes, dans lequel le deuxième fluorophore et le deuxième agent d'extinction sont liés dans la zone d'une des extrémités de l'acide nucléique indicateur.

4. Procédé selon l'une quelconque des revendications précédentes, dans lequel les étapes c1) et c2), ainsi que les étapes d1) et d2) sont réalisées successivement dans le temps en l'espace de 120 secondes, de préférence en l'espace de 60 secondes.

5. Procédé selon l'une quelconque des revendications précédentes, dans lequel les étapes c1) et. c2), ainsi que les étapes d1) et d2), sont réalisées à température ambiante.

6. Procédé selon l'une quelconque des revendications précédentes, dans lequel l'acide nucléique indicateur se présente sous la forme d'une structure en épingle à cheveux, dans laquelle l'acide nucléique indicateur présente deux branches complémentaires l'une de l'autre.

7. Procédé selon l'une quelconque des revendications précédentes, dans lequel l'acide nucléique de référence se présente sous la forme d'une structure en épingle à cheveux, dans laquelle les brins d'acide nucléique de référence présentent deux branches complémentaires l'une de l'autre.

8. Procédé selon l'une quelconque des revendications précédentes dans lequel, à l'étape c2) et/ou à l'étape d2), le marquage est exposé à la lumière d'une plage de longueurs d'onde prédéfinie.

9. Procédé selon l'une quelconque des revendications précédentes, dans lequel le marquage est appliqué sur un support réfléchissant la lumière.

10. Procédé selon l'une quelconque des revendications précédentes, dans lequel autant la solution indicatrice que la solution de référence sont appliquées sur un unique champ indicateur du marquage contenant l'acide nucléique de marquage.

11. Procédé selon l'une quelconque des revendications précédentes dans lequel, respectivement, un volume prédéfini de la solution indicatrice et/ou la solution de référence est ou sont appliqués sur le marquage.

12. Procédé selon l'une quelconque des revendications précédentes, dans lequel la première propriété escomptée est une intensité maximale prédéfinie dans une première plage de longueurs d'onde et la deuxième propriété escomptée est une intensité minimale prédéfinie dans une deuxième plage de longueurs d'onde.

13. Procédé selon l'une quelconque des revendications précédentes, dans lequel les premier et deuxième fluorophores sont identiques.

14. Procédé selon l'une quelconque des revendications précédentes, dans lequel le marquage contient, outre l'acide nucléique de marquage, au moins un autre acide nucléique.

15. Procédé selon l'une quelconque des revendications précédentes, dans lequel on utilise, comme marquage, une encre d'impression contenant l'acide nucléique de marquage et/ou l'autre acide nucléique.

16. Procédé selon l'une quelconque des revendications précédentes, dans lequel le marquage est appliqué, par un procédé d'impression, sur l'objet à marquer ou sur une étiquette de préférence autocollante.
